# EUROPÄISCHE PATENTSCHRIFT

(11) **EP 1 117 377 B1**
(45) Veröffentlichungstag und Bekanntmachung des Hinweises auf die Patenterteilung: **21.04.2004**
(21) Anmeldenummer: 99950588.6
(22) Anmeldetag: 01.10.1999
(51) Int. Cl.: A61K 7/50, A61K 7/48

(54) **KOSMETISCHE ZUBEREITUNGEN**
COSMETIC PREPARATIONS
PREPARATIONS COSMETIQUES

(30) Priorität: 10.10.1998 DE 19846773
(43) Veröffentlichungstag der Anmeldung: 25.07.2001
(73) Patentinhaber: Cognis Deutschland GmbH & Co. KG, 40589 Düsseldorf (DE)
(72) Erfinder: PRAT QUERALT, Esther, E-08328 Alella (ES); CHAZALY, Corinne, F-77400 Lagny sur Marne (FR); JACKWERTH, Bettina, D-40764 Langenfeld (DE); GASSENMEIER, Thomas, Otto, D-40229 Düsseldorf (DE)
(86) Internationale Anmeldenummer: PCT/EP1999/007273
(87) Internationale Veröffentlichungsnummer: WO 2000/021502

(56) Entgegenhaltungen:
- EP-A- 0 852 139
- EP-A- 0 879 592
- WO-A-99/39690
- DE-A- 19 652 300
- DE-C- 19 651 447
- DE-C- 19 652 302
- DE-C- 19 732 015

## Beschreibung

### Gebiet der Erfindung

Die Erfindung befindet sich auf dem Gebiet kationischer Tenside und betrifft ölhaltige kosmetische Zubereitungen mit einem Gehalt an speziellen Esterquats und niederen Alkoholen.

### Stand der Technik

Kationische Tenside vom Typ der Esterquats werden in der Kosmetik schon seit einiger Zeit für die Haaravivage eingesetzt. Wegen ihrer guten sensorischen Eigenschaften finden diese Stoffe auch zunehmend Eingang in die Hautkosmetik. Kosmetische Emulsionen, die Esterquats nach dem Stand der Technik als Avivagemittel oder kationische Emulgatoren enthalten, sind jedoch in anwendungstechnischer Hinsicht nicht völlig befriedigend. So wird von Verbrauchern beispielsweise bemängelt, daß die Mittel einen öligen Rückstand hinterlassen, sich nicht rasch genug verteilen und schneller einziehen sollten.

In diesem Zusammenhang sei auf die Deutsche Patentschrift **DE-C1 4308794** (Henkel) hingewiesen, aus der ein Verfahren zur Herstellung fester Esterquats bekannt ist, bei dem man die Quatemierung von Triethanolaminestem in Gegenwart von geeigneten Dispergatoren, vorzugsweise Fettalkoholen, durchführt. In der **DE-C1 4335782** (Henkel) wird ferner vorgeschlagen, die Quatemierung von Triethanolaminfettsäureestern in Gegenwart von Polyolen, beispielsweise Glycerin, Ethylenglycol, Partialglyceriden und dergleichen durchzuführen, um die Verwendung von Isopropylalkohol als brennbarem Lösemittel zu vermeiden.

Die Aufgabe der vorliegenden Erfindung hat folglich darin bestanden, ölhaltige kosmetische Zubereitungen mit einem Gehalt an kationischen Tensiden vom Esterquat-Typ zur Verfügung zu stellen, die sich durch verbesserte sensorische Eigenschaften und insbesondere durch rasches Verteilen und rückstandsfreies, schnelles Einziehen auszeichnen.

### Beschreibung der Erfindung

Gegenstand der Erfindung sind kosmetische Zubereitungen, enthaltend
(a) Esterquats, deren Estergruppen sich von C₈-C₁₈ Kokosfettsäuren ableiten,
(b) Ölkörper und
(c) Alkohole mit 1 bis 6 Kohlenstoffatomen, ausgewählt aus der Gruppe, die gebildet wird von Ethanol, Ethylenglycol Propylenglycol, glycerin sowie deren gemischen.

Überraschenderweise wurde gefunden, daß ölhaltige kosmetische Zubereitungen, insbesondere Haut- und Haarpflegemittel in Emulsionsform, die Esterquats auf Basis von technischen Kokosfettsäuren zusammen mit niederen Alkoholen, vorzugsweise Propylenglycol, enthalten, Haut und Haaren einen besonders angenehmen Griff verleihen, keine Rückstände hinterlassen sowie sich schnell spreiten und rasch einziehen. Die Mittel zeigen sich daher Zubereitungen mit konventionellen Esterquats, die überwiegend auf der Grundlage von längerkettigeren Palm- und Talgfettsäuren hergestellt werden, deutlich überlegen.

### Esterquats

Unter der Bezeichnung "Esterquats" werden im allgemeinen quatemierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quatemiert. Übersichten zu diesem Thema sind beispielsweise von R.Puchta et al. in **Tens.Surf.Det.,** 30, **186** (**1993**), M.Brock in **Tens.Surf.Det. 30, 394** (**1993**), R.Lagerman et al. in **J.Am.Oil.Chem.Soc.,** 71, **97** (**1994**) sowie I.Shapiro in **Cosm.Toil. 109, 77** (**1994**) erschienen.

Die die Komponente (a) bildenden Esterquats folgen beispielsweise der Formel (I), 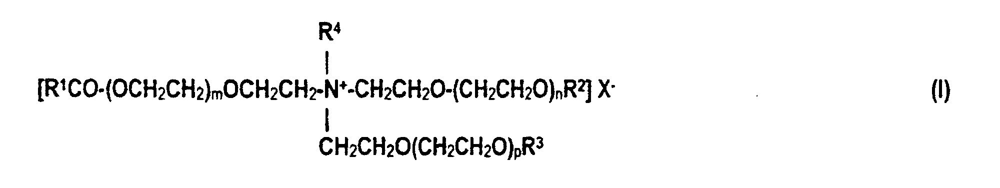 in der R¹CO für einen Kokosacylrest mit 8 bis 18, vorzugsweise 12 bis 18 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Zur Herstellung der quatemierten Ester können die Kokosfettsäuren und das Triethanolamin im molaren Verhältnis von
1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestem mit einem durchschnittlichen Veresterungsgrad von 1,5 bis 1,9 dar und leiten sich von technischer C_{12/18}-Kokosfettsäure (Iodzahl 0 bis 40) ab. Aus anwendungstechnischer Sicht haben sich quatemierte Fettsäuretriethanolaminestersalze der Formel (I) als besonders vorteilhaft erwiesen, in der R¹CO für einen Kokosacylrest mit 12 bis 18 Kohlenstoffatomen, R² für R¹CO, R³ für Wasserstoff, R⁴ für eine Methylgruppe, m, n und p für 0 und X für Methylsulfat steht.

Als weitere Gruppe geeigneter Esterquats kommen ferner quatemierte Estersalze von Kokosfettsäuren mit Diethanolalkylaminen der Formel (II) in Betracht, 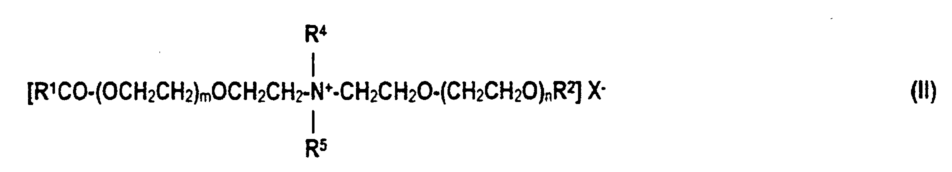 in der R¹CO für einen Kokosacylrest mit 8 bis 18, vorzugsweise 12 bis 18 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Als dritte Gruppe geeigneter Esterquats sind schließlich die quatemierten Estersalze von Kokosfettsäuren mit 1,2-Dihydroxypropyldialkylaminen der Formel (III) zu nennen, 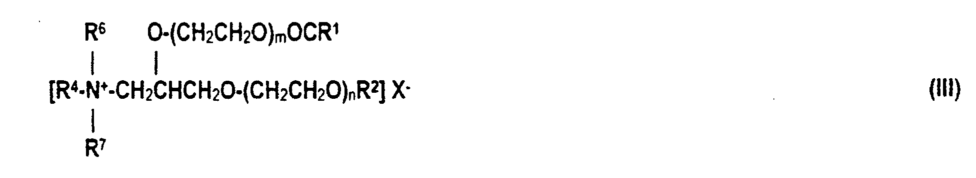 in der R¹CO für einen Kokosacylrest mit 8 bis 18, vorzugsweise 12 bis 18 Kohlenstoffatomen, R² für Wasserstoff oder R¹CO, R⁴, R⁶ und R⁷ unabhängig voneinander für Alkylreste mit 1 bis 4 Kohlenstoff-atomen, m und n in Summe für 0 oder Zahlen von 1 bis 12 und X für Halogenid, Alkylsulfat oder Alkylphosphat steht. Hinsichtlich der Auswahl des optimalen Veresterungsgrades gelten die für (I) genannten Beispiele auch für die Esterquats der Formeln (II) und (III).

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalko-holcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht.

### Alkohole

Die Auswahl der Alkohole richtet sich nach deren Verträglichkeit in kosmetischen Zubereitungen. Vorzugsweise werden daher Ethanol, Ethylenglycol und insbesondere Propylenglycol Verwendung finden. In einer bevorzugten Ausführungsform der Erfindung können technische Mischungen der Kokosesterquats mit den Alkoholen eingesetzt werden. Hierzu werden Alkanolaminfettsäureester in Gegenwart von solchen Mengen Ethanol, Ethylenglycol, Propylenglycol, Glycerin oder deren Gemischen mit Alkylierungsmitteln umsetzt, daß sich ein Gewichtsverhältnis Esterquat : Lösemittel von 95 : 5 bis 75 : 25 und vorzugsweise 90 : 10 bis 80 : 20 ergibt. Aus anwendungstechnischer Sicht ist der Einsatz von Propylenglycol sowie von Gemischen aus Ethanol und Propylenglycol im Gewichtsverhältnis 10 : 90 bis 90 : 10 und insbesondere 75 : 25 bis 25 : 75 bevorzugt. Die Veresterung und Quatemierung kann dabei in an sich bekannter Weise durchgeführt werden, wie dies beispielsweise ausführlich in den Druckschriften **DE-C1 4308794** und **DE-C1 4335782** (Henkel) beschrieben wird. Der besondere Vorteil des Einsatzes derartiger Mischungen liegt darin, daß sie auch in konzentrierter Form wasserklar sind und kalt eingearbeitet werden können.

### Kosmetische Zubereitungen

Wie schon eingangs erläutert, stellen die kosmetischen Zubereitungen der vorliegenden Erfindung üblicherweise Emulsionen dar, wobei es sich sowohl um W/O- als auch O/W-Typen handeln kann; auch multiple Emulsionen vom W/O/W- oder O/W/O-Typ kommen in Frage. In einer bevorzugten Ausführungsform der Erfindung können die Zubereitungen folgende Zusammensetzung aufweisen:
(a) 0,1 bis 25, vorzugsweise 5 bis 15 Gew.-% Kokosesterquats,
(b) 0,5 bis 90, vorzugsweise 5 bis 50 Gew.-% Ölkörper und
(c) 0,01 bis 10, vorzugsweise 0,1 bis 1 Gew.-% Alkohole,
mit der Maßgabe, daß sich die Mengenangaben mit Wasser und gegebenenfalls weiteren Inhaltsstoffen zu 100 Gew.-% ergänzen. Dabei ist es besonders bevorzugt, die Komponenten (a) und (b) in einem Gewichtsverhältnis von 80 : 20 bis 90 : 10 einzusetzen.

### Gewerbliche Anwendbarkeit

Die erfindungsgemäßen Zubereitungen, wie beispielsweise Haarkuren, Haarconditioner, Haarfärbeemulsionen, Körperreinigungs- und -pflegemittel, Sonnenschutzcremes, -lotionen oder -salben sowie Make-ups und andere dekorative Kosmetikprodukte, können ferner als weitere Hilfs- und Zusatzstoffe milde Tenside, Emulgatoren, Überfettungsmittel, Perlglanzwachse, Stabilisatoren, Konsistenzgeber, Verdickungsmittel, Polymere, Siliconverbindungen, biogene Wirkstoffe, Deowirkstoffe, Antischuppenmittel, Filmbildner, Konservierungsmittel, Hydrotrope, Solubilisatoren, UV-Lichtschutzfaktoren, Antioxidantien, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpoly-glycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 2024051** als Rückfettungsmittel für kosmetische Zubereitungen bekannt.

C_{8/18}-Alkylmono- und -oligoglycoside, ihre Hersteliung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampho-lytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.
Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologen-verteilung oder Alkylotigoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrroli-don/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Eine detaillierte Übersicht über geeignete flüchtige Silicone findet sich zudem von Todd et al. in **Cosm.Toil. 91, 27** (**1976**).

Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säu-ren, Aminosäuren, Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc.Cosm.Chem. 24, 281 (1973)**]. Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531 (1975)].** Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düssel-dorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95** (**1993**) entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylben-zyliden)campher wie in der **EP-B1 0693471** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäure-propylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylben-zylester, Salicylsäurehomomenthylester;
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP-A1 0818450** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP-B1 0694521** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sul-fonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoyl-methan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester ) sowie deren Salze, Dilaurylthiodipropionat, Distearylthiodipropional, Thiodipropionsäure und deren Derivate (Ester, Ether, Peptide, Lipide, Nukleotide, Nuk-leoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fett-säuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxy-anisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättem (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxy-ethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiver-öl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damas-cone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Herstellbeispiel 1.** In einem 1,5-I-Dreihalskolben mit Rührer, Tropftrichter und Destillationsaufsatz wurden 828 g (4 Mol) Kokosfettsäure und 1,2 g Hypophosphorsäure (50 Gew.-%ig) vorgelegt und auf 70°C erhitzt. Bei einem verminderten Druck von 30 mbar wurden portionsweise 363 g (2,4 Mol) Triethanolamin zugetropft und dabei die Temperatur bis auf 160°C gesteigert. Nach dem Ende der Zugabe wurde die Reaktionsmischung noch weitere 2 h bei 2 mbar gerührt, bis kein weiteres Reaktionswasser abgeschieden wurde und die Säurezahl einen Wert unterhalb von 5 mg KOH/g erreicht hatte. Anschließend wurden 400 g (0,86 Mol) des hergestellten Esters in einen zweiten Dreihalskolben überführt und bei 50°C in 126 g Propylenglycol gelöst. Anschließend wurden portionsweise 104 g (0,83 Mol) Dimethylsulfat zugetropft und die Mischung 4 h bei 65°C gerührt. Es wurde eine bei 20°C klare niedrigviskose Lösung erhalten, die 80 Gew.-% Esterquat und 20 Gew.-% Propylenglycol enthielt.

Die folgende Tabelle 1 enthält eine Reihe von Beispielrezepturen. Unter der Bezeichnung Dehyquart L 80 ist dabei die nach Herstellbeispiel H1 erhaltene Mischung zu verstehen.

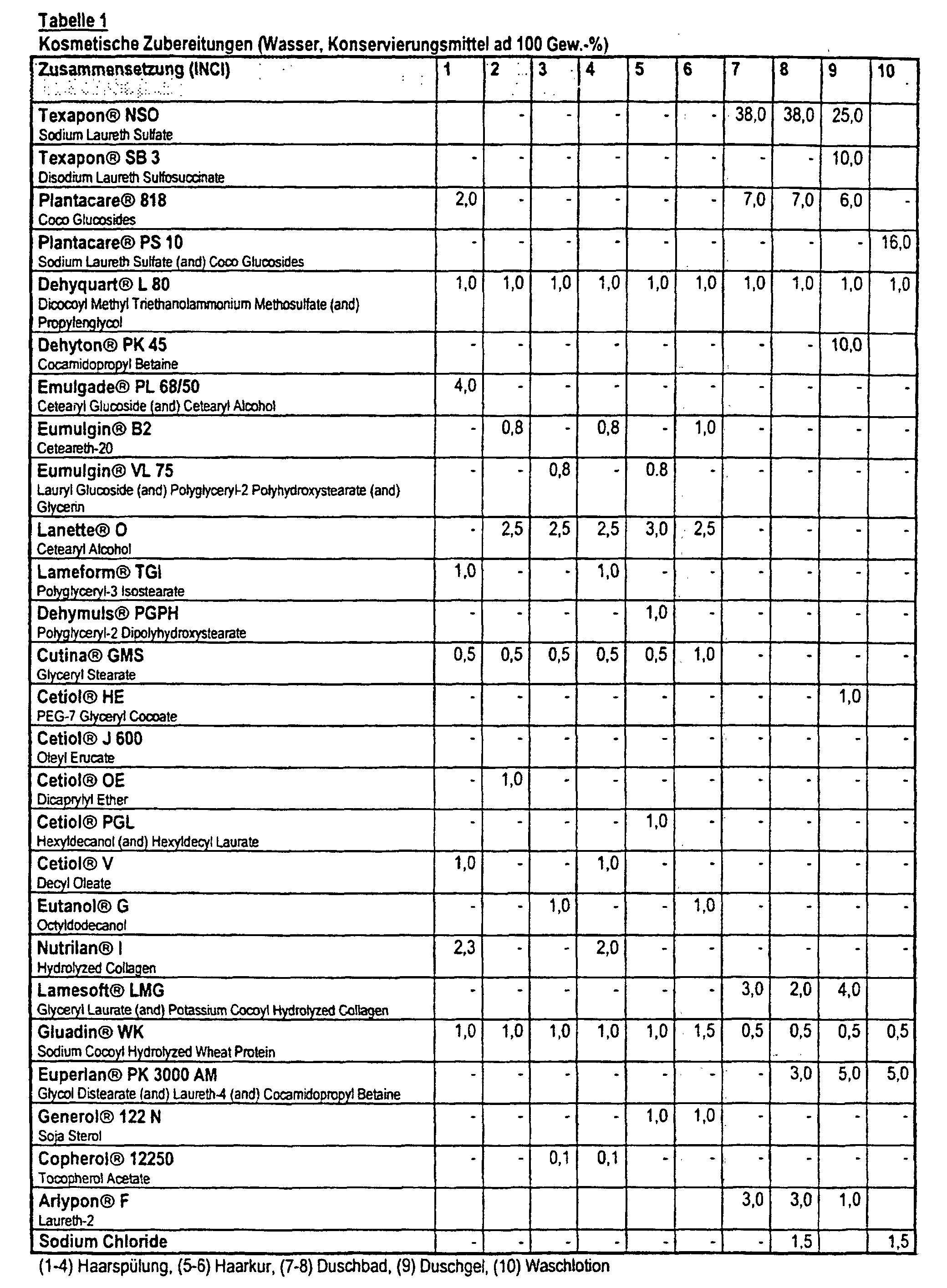

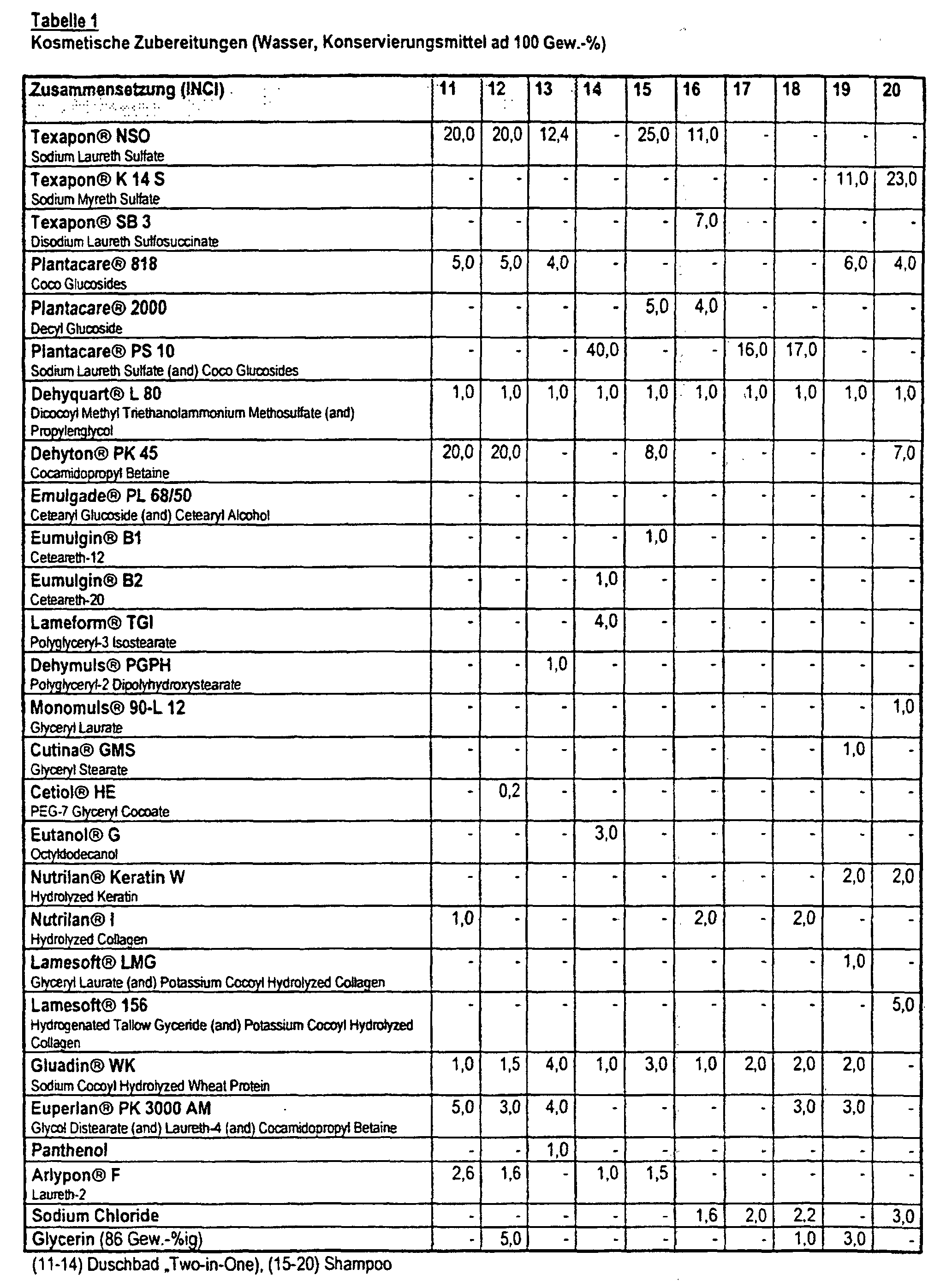

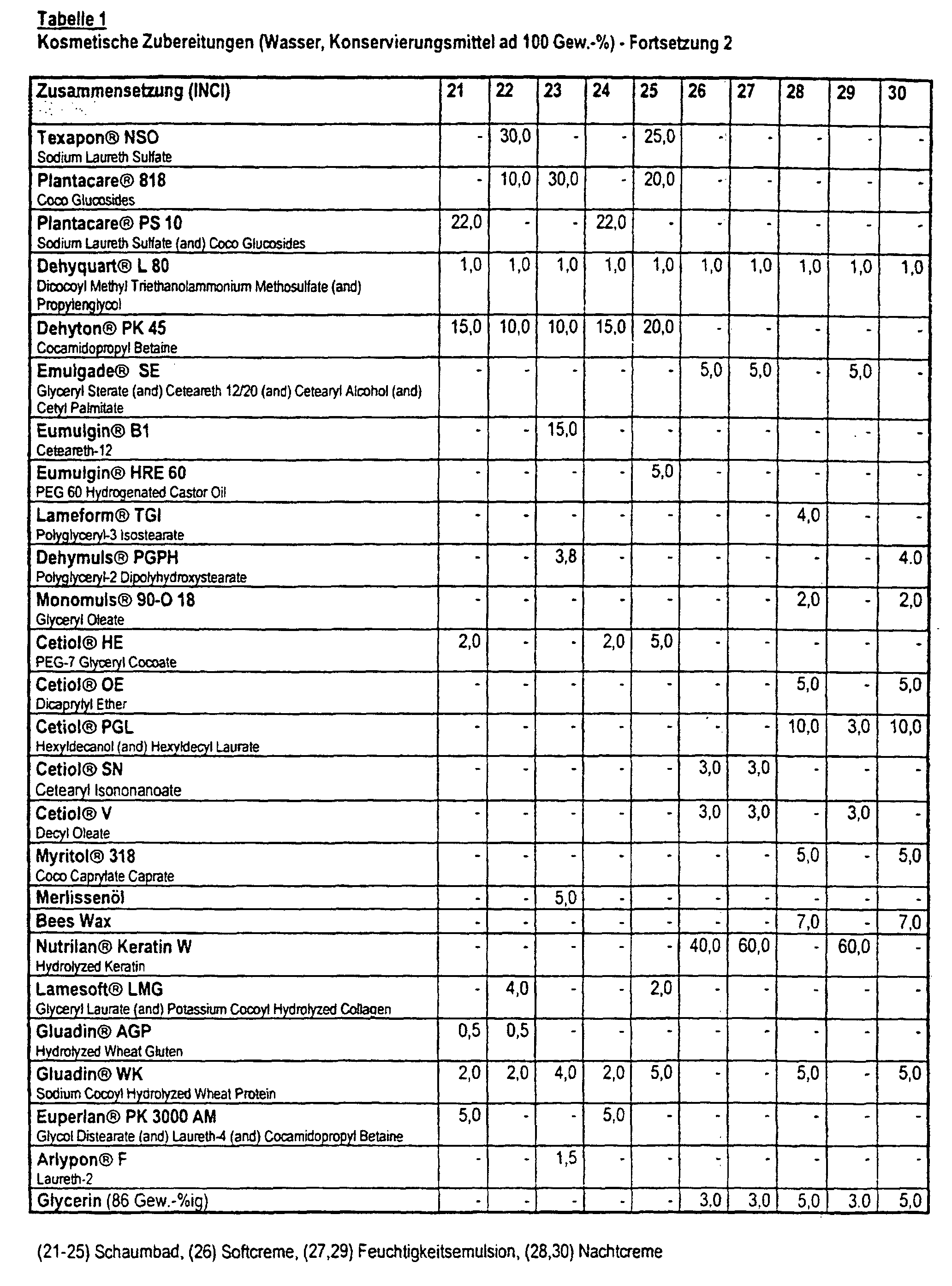

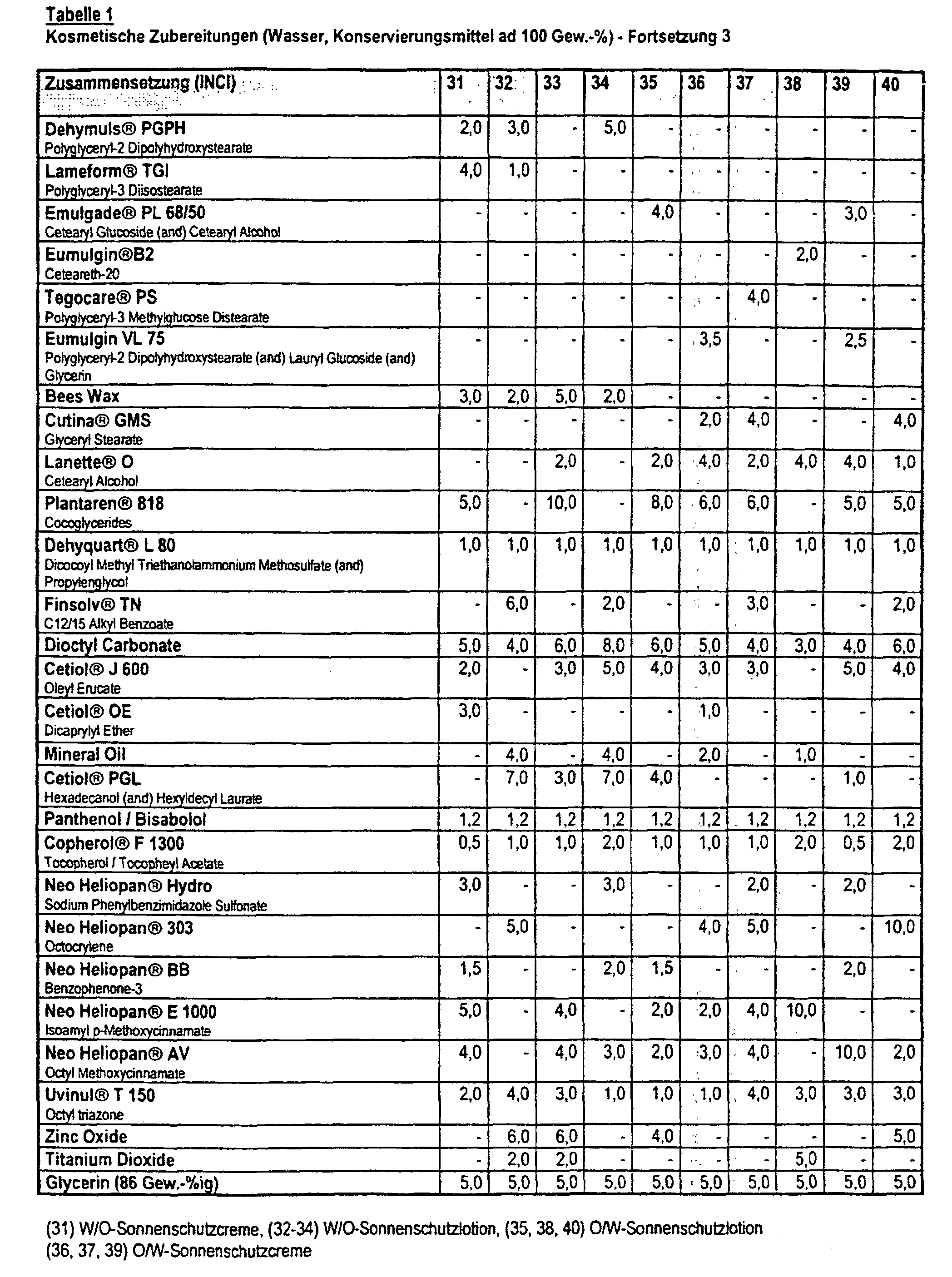

## Patentansprüche

1. Kosmetische Zubereitungen, enthaltend
(a) Esterquats, deren Estergruppen sich von C₈-C₁₈ Kokosfettsäuren ableiten,
(b) Ölkörper und
(c) Alkohole mit 1 bis 6 Kohlenstoffatomen ausgewählt aus der Gruppe, die gebildet wird von Ethanol, Ethylenglycol, Propylenglycol, Glycerin sowie deren Gemischen.

2. Zubereitungen nach Anspruch 1, **dadurch gekennzeichnet, dass** sie Esterquats der Formel **(I)** enthalten, 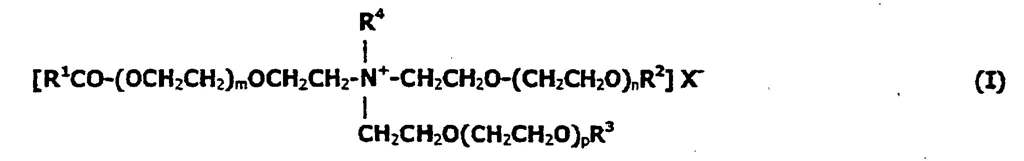 in der R¹CO für einen Kokosacylrest mit 8 bis 18 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Zubereitungen nach Anspruch 1, **dadurch gekennzeichnet, dass** sie Esterquats der Formel **(II)** enthalten, 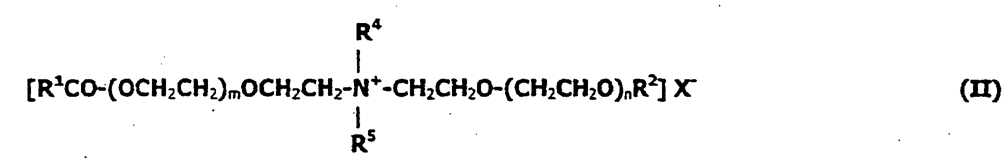 in der R¹CO für einen Kokosacylrest mit 8 bis 18 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Zubereitungen nach Anspruch 1, **dadurch gekennzeichnet, dass** sie Esterquats der Formel **(III)** enthalten, 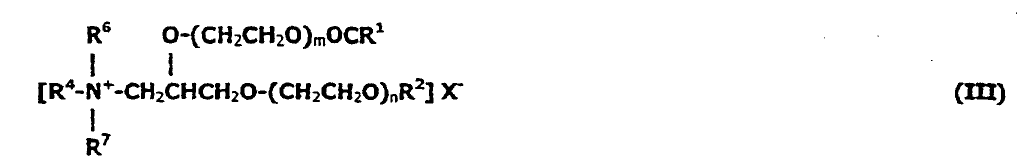 in der R¹CO für einen Kokosacylrest mit 8 bis 18 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht

5. Zubereitungen nach mindestens einem der Ansprüche 1 bis 4, **dadurch gekennzeichnet, dass** sie Ölkörper enthalten, die ausgewählt sind aus der Gruppe, die gebildet wird von Guerbetalkoholen auf Basis von Fettalkoholen mit 6 bis 18, vorzugsweise 8 bis 10 Kohlenstoffatomen, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, insbesondere 2-Ethylhexanol, Estern von Hydroxycarbonsäuren mit linearen oder verzweigten C₆-C₂₂-Fettalkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estern von C₆-C₂₂-Fettalkoholen und/oder Guerbetalkoholen mit aromatischen Carbonsäuren, Estern von C₂-C₁₂-Dicarbonsäuren mit linearen oder verzweigten Alkoholen mit 1 bis 22 Kohlen-stoffatomen oder Polyolen mit 2 bis 10 Kohlenstoffatomen und 2 bis 6 Hydroxylgruppen, pflanzliche Ölen, verzweigten primären Alkoholen, substituierten Cydohexanen, linearen und verzweigten C₆-C₂₂-Fettalkoholcarbonaten, Guerbetcarbonaten, Estern der Benzoesäure mit linearen und/oder verzweigten C₆-C₂₂-Alkoholen, linearen oder verzweigten, symmetrischen oder unsymmetrischen Dialkylethem mit 6 bis 22 Kohlenstoffatomen pro Alkylgruppe, Ringöffnungs-produkten von epoxidierten Fettsäureestern mit Polyolen, Siliconölen und/oder aliphatischen bzw. naphthenischen Kohlenwasserstoffe.

6. Zubereitungen nach mindestens einem der Ansprüche 1 bis 5, **dadurch gekennzeichnet, dass** sie Mischungen von Esterquats und Alkoholen enthalten, die auf direktem Wege durch Alkylierung von Alkanolaminfettsäureestern in den Alkoholen erhalten werden.

7. Zubereitungen nach mindestens einem der Ansprüche 1 bis 6, **dadurch gekennzeichnet, dass** sie
(a) 0,1 bis 25 Gew.-% Esterquats,
(b) 0,5 bis 90 Gew.-% Ölkörper und
(c) 0,01 bis 10 Gew.-% Alkohole,
mit der Maßgabe enthalten, dass sich die Mengenangaben mit Wasser und gegebenenfalls weiteren Inhaltsstoffen zu 100 Gew.-% ergänzen.

8. Zubereitungen nach mindestens einem der Ansprüche 1 bis 7, **dadurch gekennzeichnet, dass** sie die Esterquats und die Alkohole in einem Gewichtsverhältnis von 80 : 20 bis 90 : 10 enthalten.

## Claims

1. Cosmetic preparations containing
(a) esterquats of which the ester groups derive from C₈₋₁₈ cocofatty acids,
(b) oil components and
(c) alcohols containing 1 to 6 carbon atoms selected from the group consisting of ethanol, ethylene glycol, propylene glycol, glyerol and mixtures thereof.

2. Preparations as claimed in claim 1, **characterized in that** they contain esterquats corresponding to formula (I): 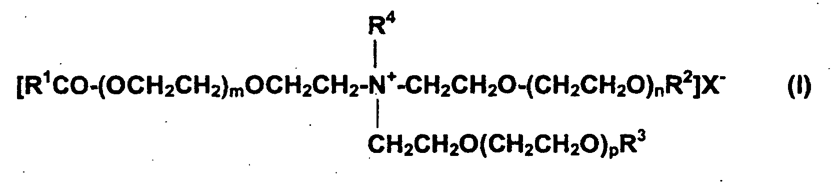 in which R¹CO is a cocoacyl group containing 8 to 18 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate.

3. Preparations as claimed in claim 1, **characterized in that** they contain esterquats corresponding to formula (II): 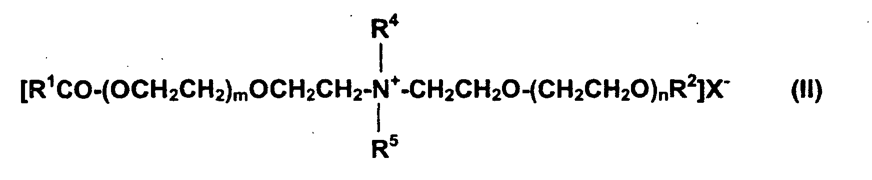 in which R¹CO is a cocoacyl group containing 8 to 18 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate.

4. Preparations as claimed in claim 1, **characterized in that** they contain esterquats corresponding to formula (III): 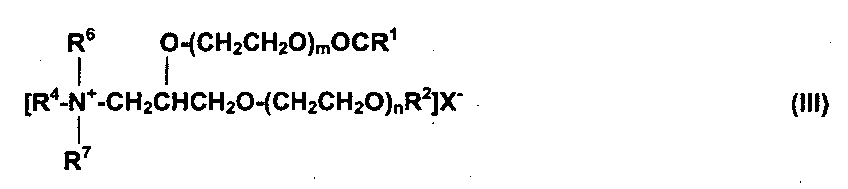 in which R¹CO, is a cocoacyl group containing 8 to 18 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate.

5. Preparations as claimed in at least one of claims 1 to 4, **characterized in that** they contain oil components selected from the group consisting of Guerbet alcohols based on fatty alcohols containing 6 to 18 and preferably 8 to 10 carbon atoms, esters of linear C₆₋₂₂ fatty acids with linear C₆₋₂₂ fatty alcohols, esters of branched C₆₋₁₃ carboxylic acids with linear C₆₋₂₂ fatty alcohols, esters of linear C₆₋₂₂ fatty acids with branched alcohols, more particularly 2-ethyl hexanol, esters of hydroxycarboxylic acids with linear or branched C₆₋₂₂ fatty alcohols, esters of linear and/or branched fatty acids with polyhydric alcohols and/or Guerbet alcohols, triglycerides based on C₆₋₁₀ fatty acids, liquid mono-/di-/triglyceride mixtures based on C₆₋₁₈ fatty acids, esters of C₆₋₂₂ fatty alcohols and/or Guerbet alcohols with aromatic carboxylic acids, esters of C₂₋₁₂ dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear and branched C₆₋₂₂ fatty alcohol carbonates, Guerbet carbonates, esters of benzoic acid with linear and/or branched C₆₋₂₂ alcohols, linear or branched, symmetrical or nonsymmetrical dialkyl ethers containing 6 to 22 carbon atoms per alkyl group, ring opening products of epoxidized fatty acid esters with polyols, silicone oils and/or aliphatic or naphthenic hydrocarbons.

6. Preparations as claimed in at least one of claims 1 to 5, **characterized in that** they contain mixtures of esterquats and alcohols directly obtained by alkylation of alkanolamine fatty acid esters in the alcohols.

7. Preparations as claimed in at least one of claims 1 to 6, **characterized in that** they contain
(a) 0.1 to 25% by weight of esterquats
(b) 0.5 to 90% by weight of oil components and
(c) 0.01 to 10% by weight of alcohols,
with the proviso that the quantities shown add up to 100% by weight with water and optionally other ingredients.

8. Preparations as claimed in at least one of claims 1 to 7, **characterized in that** they contain the esterquats and the alcohols in a ratio by weight of 80:20 to 90:10.

## Revendications

1. Préparations cosmétiques contenant,
(a) des esterquats dont les groupes ester dérivent d'acides gras de Coco en C₈-C₁₈,
(b) des composés huileux, et
(c) des alcools ayant de 1 à 6 atomes de carbone choisis dans le groupe qui est formé de l'éthanol, de l'éthylèneglycol, du propylèneglycol, du glycérol ainsi que de leurs mélanges.

2. Préparations selon la revendication 1,
**caractérisées en ce qu'**
elles renferment des esterquats de formule (I) 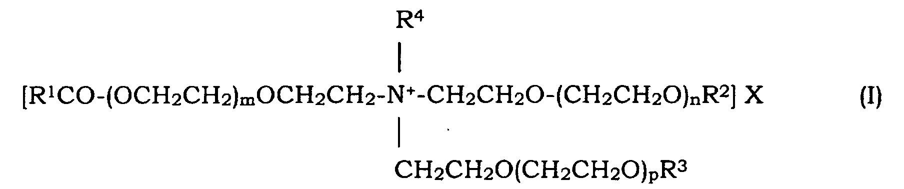 dans laquelle R¹CO représente un reste acyle de Coco ayant de 8 à 18 atomes de carbone, R² et R³ indépendamment l'un de l'autre représentent de l'hydrogène, ou R¹CO, R⁴ représente un reste alkyle ayant de 1 à 4 atomes de carbone ou un groupe (CH₂CH₂O) qH, m, n et p globalement représentent O ou des nombres allant de 1 à 12, q représente des nombres allant de 1 à 12, et X représente un halogénure, un alkylsulfate ou un alkylphosphate.

3. Préparations selon la revendication 1,
**caractérisées en ce qu'**
elles renferment des esterquats de formule (II) 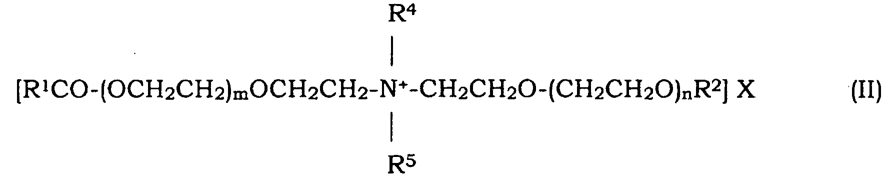 dans laquelle R¹CO représente un reste acyle de Coco ayant de 8 à 18 atomes de carbone, R² représente de l'hydrogène ou R¹CO, R⁴ et R⁵ indépendamment l'un de l'autre, représentent des restes alkyle ayant de 1 à 4 atomes de carbone, m et n globalement représentent O ou des nombres allant de 1 à 12, et X représente un halogénure, un alkylsulfate ou un alkylphosphate.

4. Préparations selon la revendication 1,
**caractérisées en ce qu'**
elles renferment des esterquats de formule (III) 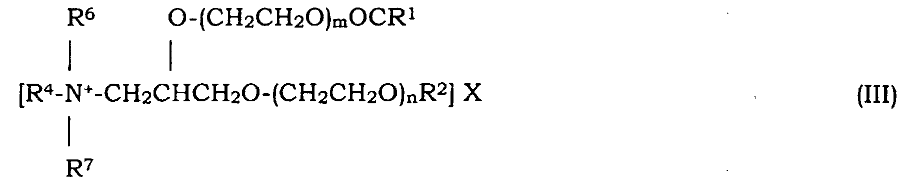 dans laquelle R¹CO représente un reste acyle de Coco ayant de 8 à 18 atomes de carbone, R² représente de l'hydrogène ou R¹CO, R⁴, R⁶ et R⁷ indépendamment les uns des autres représentent des restes alkyle ayant de 1 à 4 atomes de carbone, m et n globalement représentent O ou des nombres allant de 1 à 12, et X représente un halogénure, un alkylsulfate ou un alkylphosphate.

5. Préparations selon au moins l'une des revendications 1 à 4,
**caractérisées en ce qu'**
elles renferment des composés huileux qui sont choisis dans le groupe formé des alcools de Guerbet à base d'alcools gras ayant de 6 à 18, de préférence de 8 à 10 atomes de carbone, d'esters d'acides gras en C₆-C₂₂ linéaires avec des alcools gras en C₆-C₂₂ linéaires, des esters d'acide gras en C₆-C₁₃ ramifiés avec des alcools gras en C₆-C₂₂ linéaires, des esters d'acides gras en C₆-C₂₂ linéaires avec des alcools ramifiés, en particulier le 2-éthylhexanol, des esters d'acides carboxyliques hydroxylés avec des alcools gras linéaires ou ramifiés, en C₆-C₂₂, des esters d'acides gras linéaires et/ou ramifiés avec des alcools plurifonctionnels et/ou des alcools de Guerbet, des triglycérides à base d'acides gras en C₆-C₁₀, des mélanges liquides de mono-, di- et triglycérides à base d'acides gras en C₆-C₁₈, des esters d'alcools gras en C₆-C₂₂ et/ou d'alcools de Guerbet avec des acides aromatiques, des esters d'acides dicarboxyliques en C₂-C₁₂, avec des alcools linéaires ou ramifiés ayant de 1 à 22 atomes de carbone de carbone ou des polyols ayant de 2 à 10 atomes de carbone, et de 2 à 6 groupes hydroxyle, des huiles végétales, des alcools primaires ramifiés, des cyclohexanes substitués, des carbonates d'alcools gras en C₆-C₂₂ linéaires et ramifiés, des carbonates de Guerbet, des esters d'acide benzoïque avec des alcools linéaires et/ou ramifiés en C₆-C₂₂ ou des éthers dialkyliques linéaires ou ramifiés, symétriques ou a-symétriques ayant de 6 à 22 atomes de carbone par groupe alkyle, des produits d'ouverture de cycle d'esters d'acide gras epoxydé avec de polyols, des huiles de silicone et/ou des hydrocarbures diphatiques ou naphténiques.

6. Préparations selon au moins une des revendications 1 à 5,
**caractérisées en ce qu'**
elles renferment des mélanges d'esterquats et d'alcools qui sont obtenus par voie directe par alkylation d'esters d'acide gras et d'alkanolamine dans les alcools.

7. Préparations selon au moins une des revendications 1 à 6,
**caractérisées en ce qu'**
elles renferment de
(a) 0,1 à 25 % en poids d'esterquats
(b) 0,5 à 90 % en poids de composé huileux, et
(c) 0,01 à 10 % en poids d'alcools
avec la précision que les données en quantités se complètent à 100 % en poids avec de l'eau et éventuellement d'autres ingrédients.

8. Préparations selon au moins une des revendications 1 à 7,
**caractérisées en ce qu'**
elles renferment les esterquats et les alcools dans un rapport pondéral de 80 : 20 à 90 : 10.
